# EUROPEAN PATENT APPLICATION

(11) **EP 4 108 215 A1**
(43) Date of publication of application: **28.12.2022**
(21) Application number: 21216411.5
(22) Date of filing: 21.12.2021
(51) Int. Cl.: A61F 7/00, A41D 13/005, A41D 13/002, A41D 27/28

(54) **PORTABLE BODY SURFACE AIR COOLING METHOD AND DEVICE THEREOF**

(30) Priority: 23.06.2021 TW 110123015
(71) Applicant: CH Creative Co., Ltd., Taipei City 114 (TW)
(72) Inventor: Chang, Jui-Cheng, 204 Keelung City (TW)
(74) Representative: Cabinet Chaillot

(57) **Abstract**

A portable body surface air cooling method is disclosed. The method includes steps of compressing air with normal temperature in an environment to form compressed air, transferring the compressed air through a transfer passage, dissipating the heat of the compressed air and lowering the temperature of the compressed air in the transfer process, making the compressed air with lowered temperature continuously self-expand to reduce the pressure and lower the temperature in the transfer passage, guiding the compressed air to a terminal expansion spraying/spreading position, spraying the compressed air to the body surface of a user 4 so as to spread hot air layer on the body surface, and performing final expansion and heat absorption processes of the compressed air. A portable body surface air cooling device is also disclosed.

## Description

### 1. Field of the Invention

The present invention relates generally to a portable body surface air cooling method and a device thereof. The method mainly includes steps of compressing air with normal temperature in an environment to form compressed air with higher temperature, transferring the compressed air through a passage, dissipating the heat of the compressed air and lowering the temperature of the compressed air, making the compressed air with lowered temperature self-expand to reduce the pressure, transferring the compressed air to a terminal expansion spraying/spreading position, spraying the compressed air to the body surface of a user so as to spread hot air layer on the body surface, and performing final expansion and heat absorption processes of the compressed air so as to achieve double cooling effects.

### 2. Description of the Related Art

Various conventional easily portable cooling devices have been disclosed for partially lowering the temperature of the body of a user. For example, Taiwanese Patent Publication Nos. I290026, M425537 and 558427 disclose some of the conventional easily portable cooling devices. The conventional easily portable cooling device mainly employs a wind blowing device (a fan) to speed the air with normal temperature in an environment (without being cooled to lower the temperature) and send the air with normal temperature into an air guiding device, (that is, a clothes or a cooling passage) . The speeded airflow directly blows to the body surface of the user so as to forcedly carry away the hot flow near the body surface of the user and achieve temperature-lowing and cooling effect. However, in practical application of the above structures, the airflow is common air with normal temperature without being cooled to lower the temperature. Therefore, such conventional portable cooling device can simply force the air near the body surface to more quickly flow through the body surface so as to increase the amount of the hot flow carried away from the body surface . As a result, the conventional portable cooling device can only achieve a quite limited cooling effect. Moreover, when the airflow is driven and sent by the above wind blowing device into the narrow air guiding device (the clothes or the cooling passage), due to the wind resistance of the air passage, it is hard for the air entering the air passage to continuously smoothly flow. Therefore, the cooling effect is even more deteriorated.

In addition, Taiwanese Patent Publication Nos. M598060, M424789 and 201100030 disclose some other conventional cooling devices. All of such conventional cooling device employ an electronic cooling cell (cooling chip or thermal diode) to previously cool the air with normal temperature in an environment by means of heat exchange. Then the cooled air is guided and transferred to a position in direct or indirect contact with the body surface of a user so as to achieve temperature-lowering and cooling effect. However, the electronic cooling cell (cooling chip or thermal diode) has lower cooling efficiency per unit energy (40% to 50%). Under the limitation of the lower cooling efficiency, the electronic cooling cell can hardly provide sufficient and satisfying cooling effect for outdoor portable air-conditioning requirements necessitating higher cooling effect. Therefore, with respect to those situations in which the outdoor portable cooling device is applied and a long-term air cooling effect is required, the electronic cooling cell can hardly provide sufficient and satisfying temperature lowering and cooling effect.

Taiwanese Patent Publication No. M589457 discloses a conventional coolant-type air-conditioning system composed of a compressor, an evaporator, an expansion valve and a condenser. The conventional coolant-type air-conditioning system serves to cool the air in an environment to lower the temperature of the air. Then the cooled air is blown to the body surface of a user so as to achieve temperature-lowering and cooling effect. Such device utilizes coolant circulation to indirectly cool the air to achieve stronger cooling effect and is able to cool a great amount of air and lower the temperature of the air. However, the use of the coolant often leads to the problem of contamination of the environment or contamination of the air-conditioning set. Moreover, the entire structure of such air-conditioning system is complicated and has a huge volume so that it is inconvenient to carry and use the air-conditioning system and the cost for the air-conditioning system is very high. Furthermore, the entire system needs to consume a great amount of power for operation so that it is inconvenient for the user to carry and use the air-conditioning system and the air-conditioning system fails to meet economic benefits.

### SUMMARY OF THE INVENTION

It is therefore a primary object of the present invention to provide a portable body surface air cooling method to eliminate the shortcomings of the aforesaid various conventional portable cooling devices for partially lowering the temperature of human body. The portable body surface air cooling method of the present invention includes steps of compressing air with normal temperature in an environment to form compressed air with higher temperature, transferring the compressed air through a passage, dissipating the heat of the compressed air and lowering the temperature of the compressed air with higher temperature in the transfer process, making the compressed air with lowered temperature continuously gently self-expand to reduce the pressure and lower the temperature in the transfer passage, transferring the compressed air to a terminal expansion spraying/spreading position, directly spraying the compressed air to the body surface of a user so as to spread hot air layer on the body surface, and performing final expansion and heat absorption processes of the compressed air to achieve double cooling effects.

To achieve the above and other objects, the portable body surface air cooling device of the present invention includes a portable air compression and heat dissipation set, a portable power supply device and a compressed air transfer and terminal expansion spraying/spreading device. The portable air compression and heat dissipation set is composed of at least one air compression unit, an air heat dissipation passage set and a power unit (a motor) . The portable air compression and heat dissipation set is assembled with the portable power supply device (mobile battery set) and the compressed air transfer and terminal expansion spraying/spreading device. When the portable power supply device supplies power to the power unit to operate, the air compression unit is driven to take in the air in an environment and compress the air into compressed air with higher temperature. Thereafter, the compressed air is transferred through the air heat dissipation passage set to dissipate the heat. In the transfer process, an externally added heat exchange heat dissipation unit is further used to perform externally added heat dissipation and temperature lowering process to the compressed air. After that, in the transfer pipeline of the compressed air transfer and terminal expansion spraying/spreading device, the compressed air with lowered temperature continuously gently self-expands to reduce the pressure and lower the temperature. By means of the pushing force created when the compressed air self-expands, the compressed air is transferred to the body surface position of the user. Finally, in the position near the body surface of the user, the compressed air with fully lowered temperature and fully reduced pressure is directly sprayed to the body surface of the user to spread the hot air layer near the body surface and perform the processes of final expansion and heat absorption so as to achieve double cooling effects.

In order to more easily wear or carry the portable body surface air cooling device of the present invention on the back, the portable air compression and heat dissipation set and the portable power supply device can be placed on a wearable set carrier body (such as a waistband-type carrier body or a backpack-type carrier body). In the case that the wearable set carrier body is a backpack-type carrier body, the backpack strap and the portions of the backpack-type carrier body in contact with the back of the user can be designed with compressed air terminal expansion spraying/spreading passages, whereby the compressed air can be sprayed from the portions of the backpack-type carrier body in contact with the body surface or the clothes worn on the body surface so as to achieve cooling effect.

The present invention can be best understood through the following description and accompanying drawings, wherein:

### BRIEF DESCRIPTION OF THE DRAWINGS

Fig. 1 is a flow chart of the portable body surface air cooling device of the present invention;
Fig. 2 is a perspective view of the portable body surface air cooling device of the present invention, which is designed in accordance with the cooling method as shown in Fig. 1;
Fig. 3 is a perspective sectional view of the portable air compression and heat dissipation set of the present invention according to Fig. 2;
Fig. 4 is a sectional view of the air compression unit of the present invention according to Fig. 3, showing internal structure thereof;
Fig. 5 is a sectional view of the uninterrupted air heat dissipation passage pipeline and the radiating fins of the present invention according to Fig. 3;
Fig. 6 is a perspective view of another embodiment of the present invention according to Fig. 2, showing that the neck brace-type terminal expansion spraying/spreading unit is connected with other terminal expansion spraying/spreading units;
Fig. 7-1 is a perspective view of an embodiment of the present invention, showing that the present invention is designed with a form of both a backpack-type wearable set carrier body and a terminal expansion spraying/spreading passage;
Fig. 7-2 is a perspective view of an embodiment of the present invention, showing that the present invention is designed with a form of both a sling bag-type wearable set carrier body and a terminal expansion spraying/spreading passage;
Fig. 7-3 is a perspective view of an embodiment of the present invention, showing that the present invention is designed with a form of both a backpack-type wearable set carrier body and a terminal expansion spraying/spreading passage and connected and assembled with other terminal expansion spraying/spreading units;
Fig. 8-1 is a perspective view of the neck brace-type terminal expansion spraying/spreading unit according to Fig. 2, showing that the neck brace-type terminal expansion spraying/spreading unit is designed with a form of two stretchable segments;
Fig. 8-2 is a perspective view of the neck brace-type terminal expansion spraying/spreading unit according to Fig. 2, showing that the two stretchable segments of the neck brace-type terminal expansion spraying/spreading unit are stretched open; and
Fig. 9 is a perspective view of the armband-type terminal expansion spraying/spreading unit according to Fig. 2, showing that the processes of terminal expansion spraying/spreading and expansion heat absorption of the compressed air are performed.

### DETAILED DESCRIPTION OF THE PREFERRED EMBODIMENTS

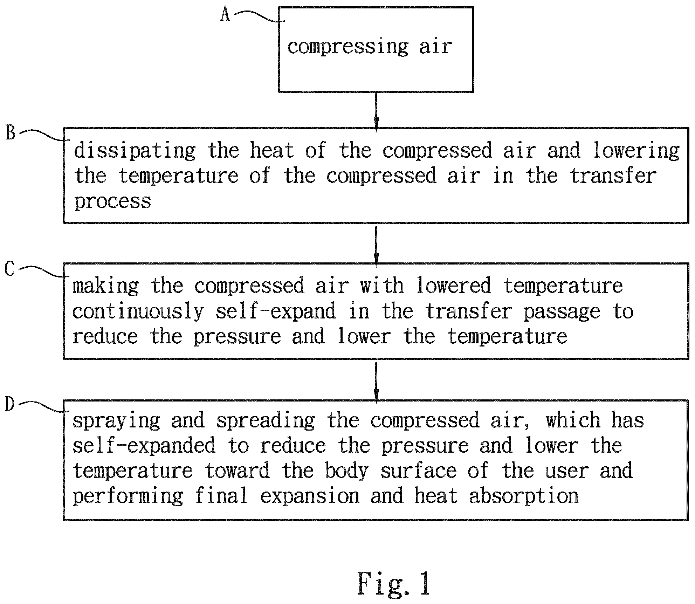

Please refer to Fig. 1. The present invention mainly provides a portable body surface air cooling method to improve the shortcomings of the aforesaid various conventional portable cooling devices for partially lowering the temperature of the body of a user. The portable body surface air cooling method of the present invention includes steps of:
(A) compressing air, the air in an environment being compressed into compressed air, which has such a physical property that in a normal environment, the compressed air can self-expand;
(B) dissipating the heat of the compressed air and lowering the temperature of the compressed air in the transfer process, the compressed air being transferred through a transfer passage, in the transfer process, the heat of the compressed air being dissipated and the temperature of the compressed air being lowered to lower the temperature of the compressed air, in the transfer process, an airflow disturbingmember (a fan or a blower) or an electronic cooling cell (a cooling chip) or the like being further used to perform externally added heat exchange operation to the transfer passage so as to speed heat dissipation and enhance heat dissipation and temperature lowering effect for the compressed air;
(C) making the compressed air with lowered temperature continuously self-expand in the transfer passage heat-insulated from outer side thereof to reduce the pressure and lower the temperature, the compressed air with lowered temperature being continuously transferred through the transfer passage, whereby in the transfer process, the compressed air can continuously self-expand to reduce the pressure and lower the temperature; and
(D) spraying and spreading the compressed air, which has self-expanded to reduce the pressure and lower the temperature toward the body surface of the user and performing final expansion and heat absorption, the compressed air that has self-expanded to reduce the pressure and lower the temperature in step C being directly sprayed and spread toward the body surface of the user so as to spread the hot air layer near the body surface, also, in the spraying/spreading process, the compressed air being made to perform final expansion and heat absorption effect so as to lower the temperature near the body surface and achieve double cooling effects.

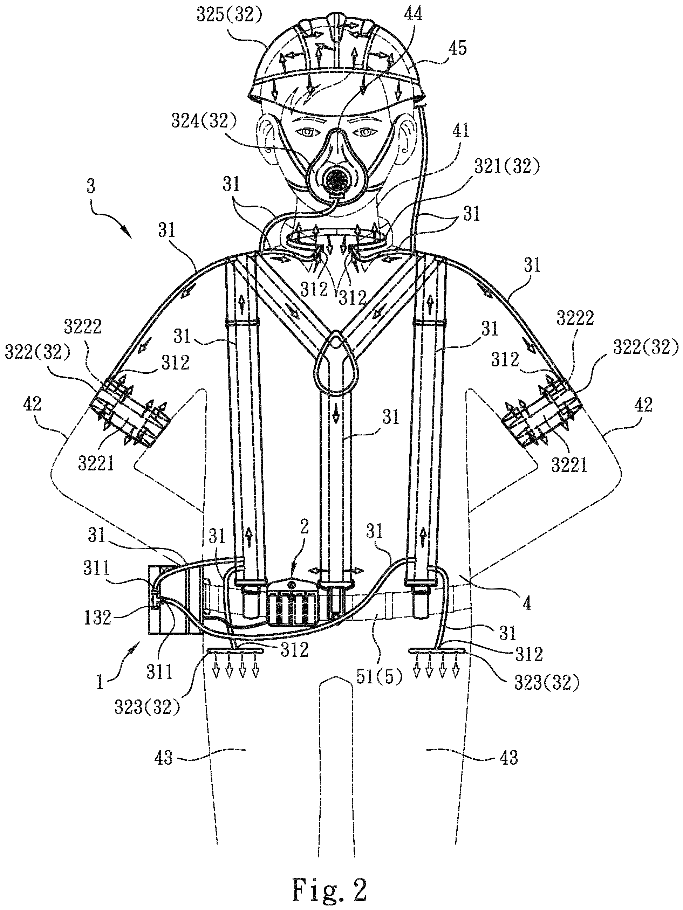

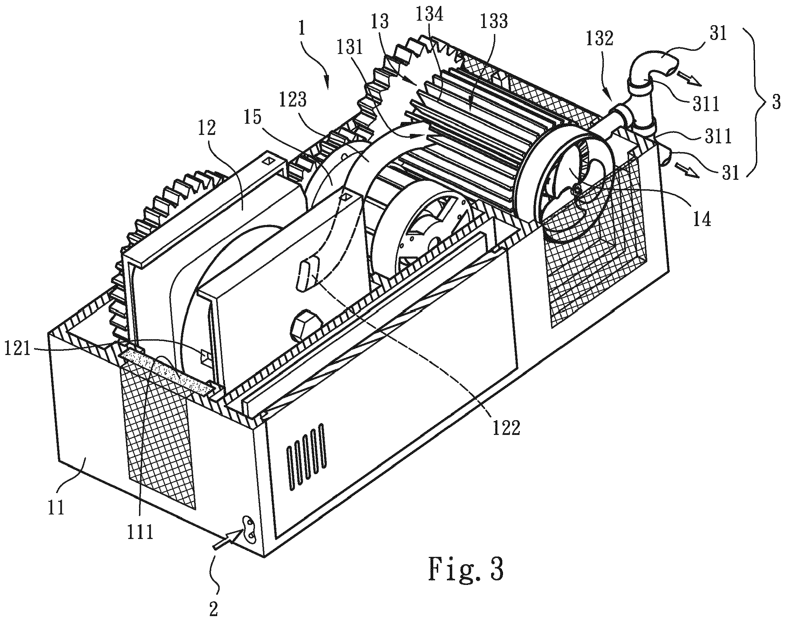

Please refer to Figs. 2 and 3. The portable body surface air cooling device of the present invention is designed in accordance with the cooling method of the present invention, mainly including a portable air compression and heat dissipation set 1, a portable power supply device 2 and a compressed air transfer and terminal expansion spraying/spreading device 3. The portable air compression and heat dissipation set 1 is composed of a case 11, an air compression unit 12, an air heat dissipation passage set 13, an externally added heat exchange heat dissipation unit 14 and a power unit 15. The air compression unit 12 has an air intake 121, a compressed air exhaustion port 122 and a compressed air conduit 123. An air filtering member 111 can be additionally disposed to filter the air taken into the air compression unit 12 in at least one of two situations before the air is taken into the air compression unit 12 and after the air is compressed. The air heat dissipation passage set 13 has an air heat dissipation passage air inlet 131 and an air heat dissipation passage air outlet 132. One of two ends of the compressed air conduit 123 is connected with the compressed air exhaustion port 122 of the air compression unit 12, while the other of the two ends of the compressed air conduit 123 is connected with the air heat dissipation passage air inlet 131 of the air heat dissipation passage set 13. The air heat dissipation passage air outlet 132 of the air heat dissipation passage set 13 is connected with the compressed air transfer and terminal expansion spraying/spreading device 3. Accordingly, after the heat of the compressed air is dissipated to lower the temperature through the air heat dissipation passage set 13, the compressed air can be further transferred to a position close to the body surface of a user 4 via the compressed air transfer and terminal expansion spraying/spreading device 3. In this case, the compressed air can be directly sprayed to the body surface of the user 4 to spread the hot air layer and perform the final process of expansion, heat absorption and temperature lowering.

According to the assembly of the above devices, when the portable power supply device 2 supplies power to the power unit 15, the power unit 15 is operated to drive the air compression unit 12. At this time, the ambient air is taken into the air compression unit 12 through the air intake 121 and compressed into compressed air with higher temperature. The compressed air with higher temperature then goes from the compressed air exhaustion port 122 into the compressed air conduit 123 and then goes from the air heat dissipation passage air inlet 131 into the air heat dissipation passage set 13. When the compressed air with higher temperature goes through the transfer passage of the air heat dissipation passage set 13, the thermal energy of higher temperature can be transmitted to the transfer passage, which transmits thermal energy to outer side to lower the temperature of the compressed air. In addition, the externally added heat exchange heat dissipation unit 14 (such as a fan, a blower, a cooling chip, a cooling water block or a cooling pipeline) is disposed around the transfer passage to speed the heat dissipation and temperature lowering process of the transfer passage. After compressed air with lowered temperature is produced, the compressed air with lowered temperature is further exhausted from the air heat dissipation passage air outlet 132 to the compressed air transfer and terminal expansion spraying/spreading device 3 connected therewith. In an air transfer pipeline 31 of the compressed air transfer and terminal expansion spraying/spreading device 3, the compressed air can automatically gently expand to reduce the pressure and lower the temperature in a condition that the compressed air is fully isolated from the affection of external ambient temperature. The air is then transferred to a position close to the body surface of the user 4. During this process, the compressed air with lowered temperature again expands to reduce the pressure so that the air pressure and temperature of the compressed air are continuously reduced to become even lower. Finally, the nearly uncompressed low-temperature compressed air is directly sprayed to the body surface of the user 4 to perform both a process of spreading the hot air layer 60 and a process of final expansion, heat absorption and temperature lowering. Accordingly, a better mobile cooling effect is achieved.

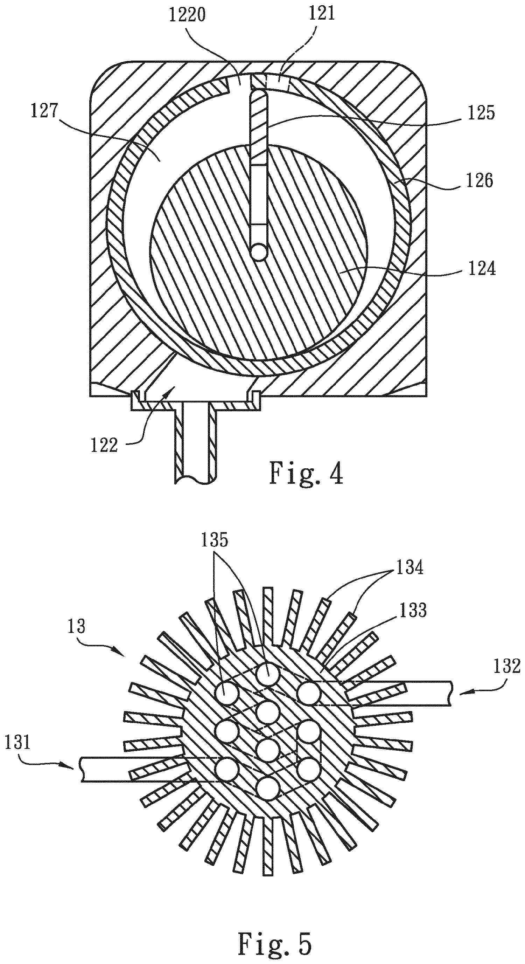

Please refer to Fig. 4, which is a sectional view showing the internal structure of the air compression unit 12 of this embodiment. The air compression unit 12 at least has a vane rotor 124, a vane 125 and a rotational vane chamber case 126. The rotational vane chamber case 126 is eccentric relative to the vane rotor 124, whereby the rotational vane chamber case 126 is formed with an internal eccentric vane chamber section 127. The eccentric vane chamber section 127 is partitioned by the vane 125 into a compression side and an intake side. An air exhaustion port 1220 is formed on the rotational vane chamber case 126 on the compression side. An air intake port 121 is formed on the rotational vane chamber case 126 on the intake side. The air enters the air compression unit 12 from the air intake port 121. After compressed, when the air exhaustion port 1220 is in communication with the compressed air exhaustion port 122, the compressed air is exhausted out of the air compression unit 12 through the compressed air exhaustion port 122.

Please refer to Fig. 5, which is a sectional view of the air heat dissipation passage set 13 of this embodiment. The air heat dissipation passage set 13 is composed of an air heat dissipation passage main body 133, radiating fins 134 and air heat dissipation passage tunnels 135 formed in the air heat dissipation passage main body 133 as the compressed air transfer passages . When the compressed air with higher temperature enters the air heat dissipation passage tunnels 135, the heat of the compressed air with higher temperature is outward transmitted to dissipate the heat through the air heat dissipation passage main body 133 and the radiating fins 134. Therefore, the temperature of the compressed air is lowered. In addition, the externally added heat exchange heat dissipation unit 14 (such as a fan, a blower, a cooling chip, a cooling water block or a cooling pipeline) is disposed around the air heat dissipation passage set 13 to enhance the heat dissipation and temperature lowering effect for the air heat dissipation passage set 13. Moreover, multiple air heat dissipation passage main body 133 and radiating fins 134 can be connected and assembled as necessary to achieve better heat dissipation effect.

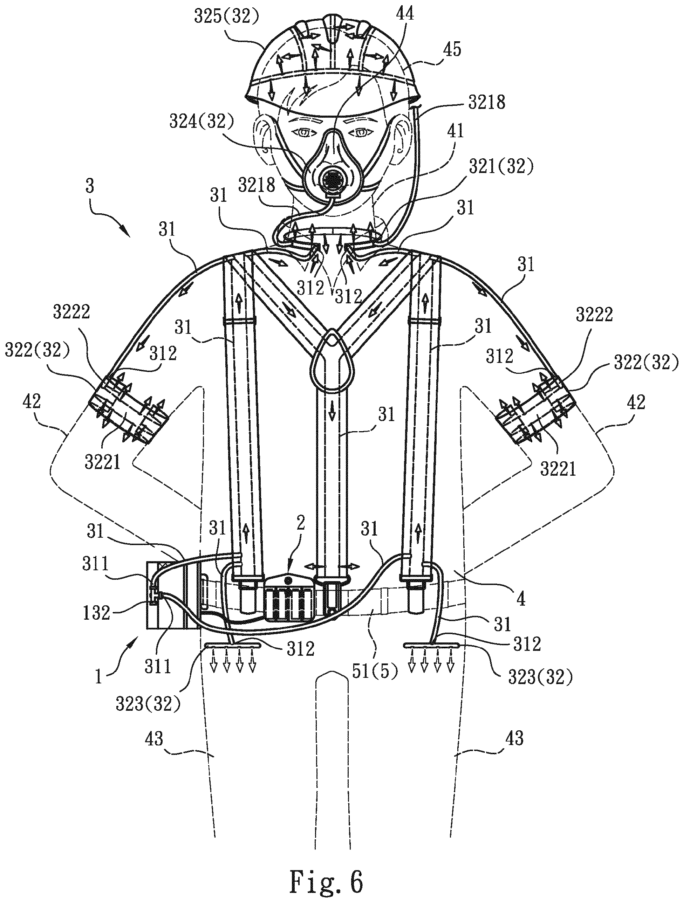

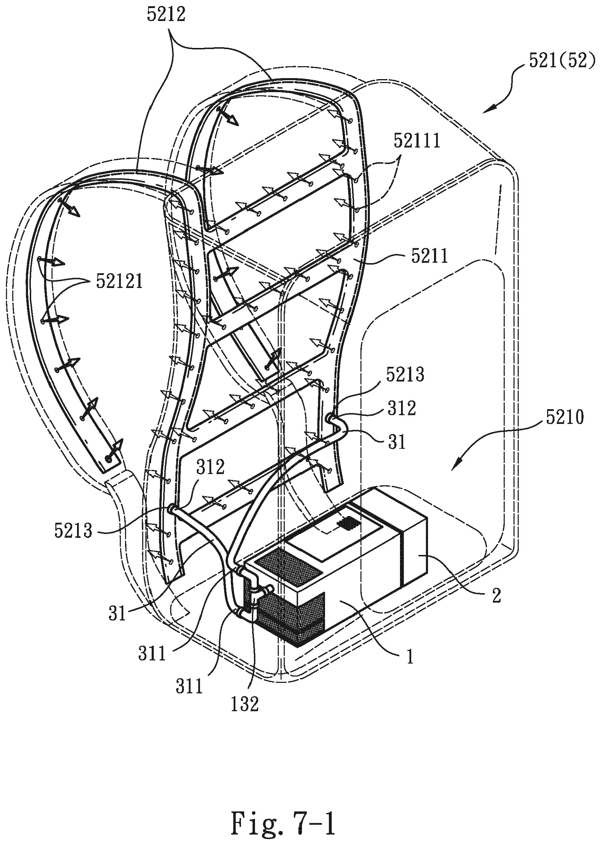

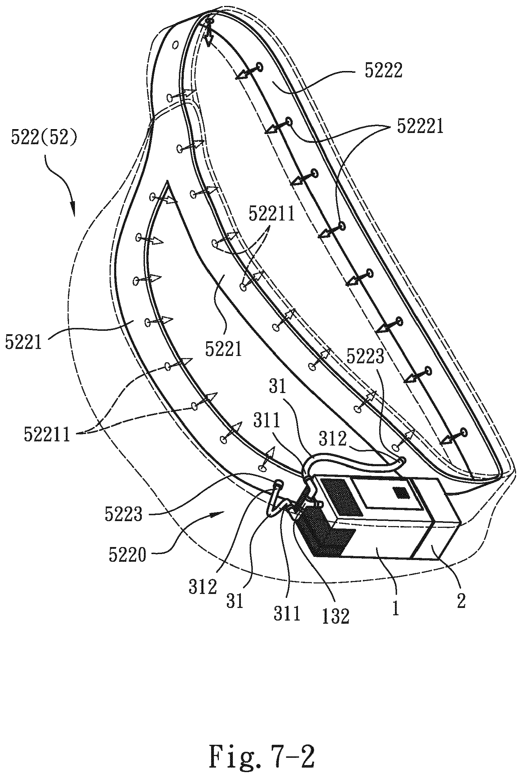

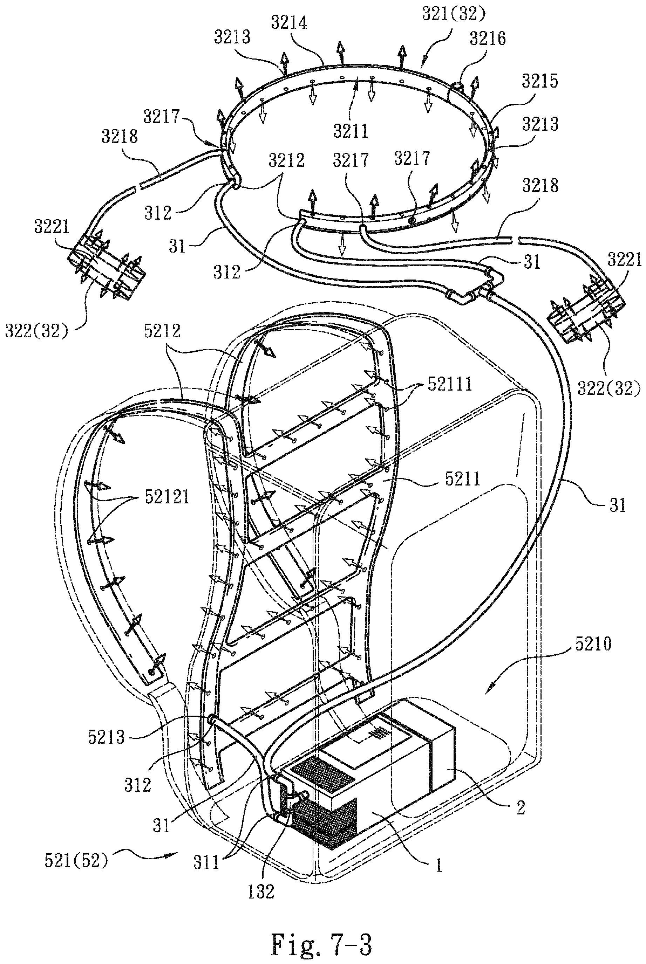

Please refer to Fig. 2. The portable air compression and heat dissipation set 1 can be directly worn and carried on the body of the user 4 by means of a wearable set carrier body 5, (for example, carried on the waist of the user 4 by means of a waistband-type carrier body 51 as shown in Figs. 2 and 6 or carried on the back of the user 4 by means of a backpack-type carrier body 52 as shown in Figs. 7-1 and 7-2 or worn on the body of the user 4 by means of a clothes-type carrier body). The portable power supply device 2 can be inbuilt in the portable air compression and heat dissipation set 1. Alternatively, the portable power supply device 2 can be an externally added device carried on or received in the wearable set carrier body 5 worn on the user 4. The compressed air transfer and terminal expansion spraying/spreading device 3 can be designed with a form, which can be attached to or connected with a vest, a clothes, a suspender wearable on the body of the user 4 or a backpack strap in contact with the back of the user 4. In this case, the compressed air transfer and terminal expansion spraying/spreading device 3 can be worn or carried on the body of the user 4 or suspended from the shoulder of the user 4. The compressed air transfer and terminal expansion spraying/spreading device 3 includes an air transfer pipeline 31 for transferring the compressed air and expansion of the compressed air. The air transfer pipeline 31 has at least one intake port 311 in communication with the air heat dissipation passage air outlet 132 of the air heat dissipation passage set 13, whereby after the compressed air passes through the air heat dissipation passage set 13 to dissipate the heat and lower the temperature, the compressed air can enter the air transfer pipeline 31 from the air heat dissipation passage air outlet 132. Then the compressed air can be further transferred in a condition that the compressed air is fully isolated from the affection of the external ambient temperature. At least one air exhaustion port 312 is disposed at the other end of the air transfer pipeline 31. Each air exhaustion port 312 is in connection and communication with a terminal expansion spraying/spreading unit 32 in a proper form. The terminal expansion spraying/spreading unit 32 can be designed with various forms in accordance with different application parts of the user 4. For example, as shown in Fig. 2, the terminal expansion spraying/spreading unit 32 at least includes a neck brace-type terminal expansion spraying/spreading unit 321 wearable on the neck 41 of the user 4, an armband-type terminal expansion spraying/spreading unit 322 wearable on the arm 42 of the user 4, a lower waist-type terminal expansion spraying/spreading unit 323 for spraying low-temperature compressed air to the limbs 43 below the waist of the user 4, a mask-type terminal expansion spraying/spreading unit 324 for the mouth and nose 44 of the user 4 to breathe and a helmet-type terminal expansion spraying/spreading unit 325 wearable on the head 45 of the user 4.

It should be especially noted that at the heat dissipation stage, no matter whether the compressed air is in the air heat dissipation passage tunnels 135 or in the air transfer pipeline 31 anterior to the respective terminal expansion spraying/spreading units 32, the portable body surface air cooling device of the present invention can be such designed that there is no intervening component such as switch valve or the like, which will interrupt or block the air transfer pipeline, and the compressed air is not input into the interior of any device with an isolating space, which then collectively dissipates the heat of the compressed air. In this case, the compressed air is directly transferred through an uninterrupted transfer passage, whereby the compressed air will naturally expand to reduce the pressure and the heat of the compressed air is dissipated to lower the temperature. Accordingly, the sizes and lengths of the air heat dissipation passage tunnels 135 and the air transfer pipeline 31 can be directly utilized to control the expansion and spreading rate and the temperature lowering speed of the compressed air so as to further control the heat dissipation and cooling effect. Therefore, the volume and weight of the present invention can be minified to enhance the portability and practical use value of the entire portable body surface air cooling device of the present invention. This is one of the primary special heat dissipation control effects of the present invention.

Please refer to Figs. 7-1 and 7-2. The backpack-type carrier body 52 includes an often seen backpack-type carrier body 521 and an often seen sling bag-type carrier body 522. The backpack-type carrier body 52 has internal spaces 5210, 5220 for receiving the portable air compression and heat dissipation set 1 and the portable power supply device 2 as the waistband-type carrier body 51. In addition, the backpack-type carrier body 521 and the sling bag-type carrier body 522 are designed with backing face air transfer passages 5211, 5221 and back strap air transfer passages 5212, 5222 in those parts in contact with the user 4. Multiple air spraying/spreading orifices 52111, 52121, 52211, 52221 are formed on the backing face air transfer passages 5211, 5221 and the back strap air transfer passages 5212, 5222. The compressed air output from the portable air compression and heat dissipation set 1 can be transferred from the intake ports 5213, 5223 to the backing face air transfer passages 5211, 5221 and back strap air transfer passages 5212, 5222. Then the compressed air is sprayed and spread from the multiple air spraying/spreading orifices 52111, 52121, 52211, 52221 onto the body surface of the user 4 or the clothes on outer side of the body surface to perform final spraying/spreading and expansion so as to blow toward the body surface to absorb the heat of the body surface and lower the temperature of the body surface and cool the body surface. In the case that the body surface is separated from the backing face air transfer passages 5211, 5221 and back strap air transfer passages 5212, 5222 by the clothes, the cooling effect will be less than direct action on the body surface. However, it is quite convenient to use the backpack-type carrier body 52. The backpack-type carrier body 52 can be easily put on or taken off.

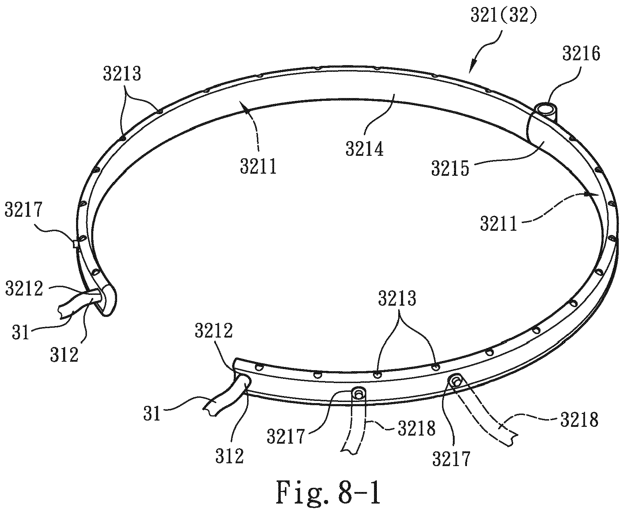

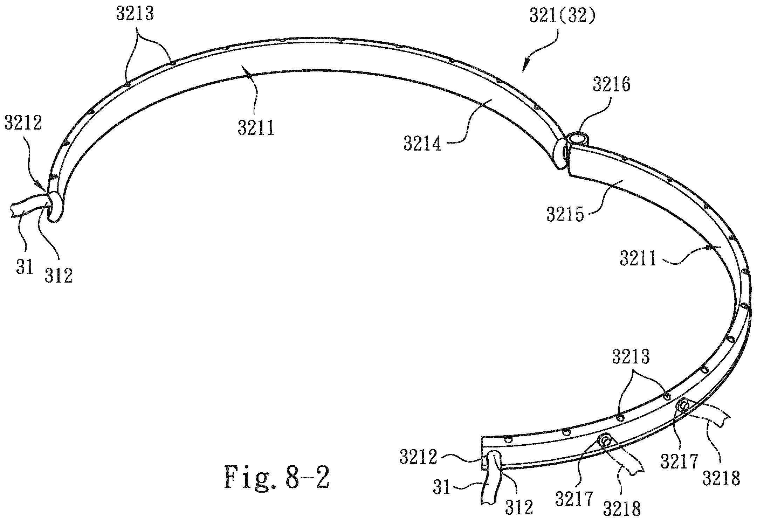

Please refer to Figs. 8-1 and 8-2. The neck brace-type terminal expansion spraying/spreading unit 321 can be a hollow annular structure body (321) exceeding a semicircle. The hollow annular structure body (321) can be snugly fitted around the neck 41 of the user 4. The hollow annular structure body (321) is formed with an internal communication passage 3211. In addition, the hollow annular structure body (321) has air incoming holes 3212 in communication with the internal communication passage 3211. The air incoming holes 3212 are in connection and communication with the air exhaustion port 312 of the air transfer pipeline 31. Multiple air spraying/spreading orifices 3213 are disposed on a sidewall of the hollow annular structure body (321) in communication with the internal communication passage 3211. The air spraying/spreading orifices 3213 are directed to the neck 41 of the user 4 or the body surface of the body part below the neck 41 of the user 4, whereby the air in the internal communication passage 3211 can be sprayed and spread from the air spraying/spreading orifices 3213 toward the body surface around the neck 41 of the user 4 to achieve the effects of final expansion, heat absorption and temperature lowering. In order to facilitate wear, the hollow annular structure body (321) can be divided into at least two stretchable segments 3214, 3215, which are pivotally connected via a pivotal connection section 3216. Accordingly, when wearing the hollow annular structure body (321), the pivotal connection angle between the two stretchable segments 3214, 3215 can be adjusted, whereby the user 4 can conveniently wear the hollow annular structure body (321) and adjust the stretchable segments 3214, 3215 to a comfortable angle (as shown in Fig. 8-2) .

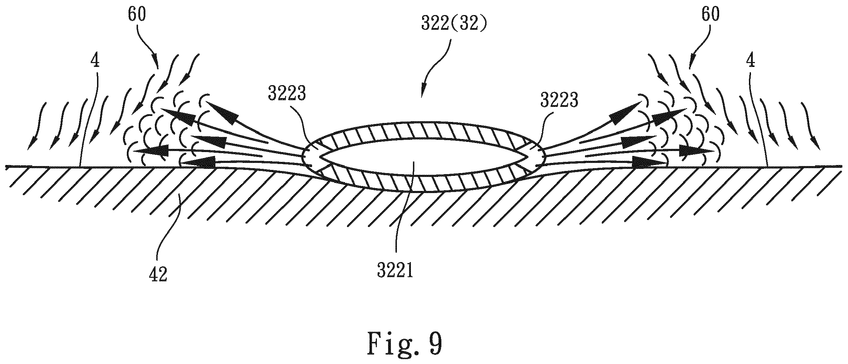

Please further refer to Figs. 2 and 9. The armband-type terminal expansion spraying/spreading unit 322 can be an annular band body snugly wearable on the arm 42 of the user 4. The annular band body is formed with an internal hollow air passage 3221. In addition, the annular band body has an air incoming hole 3222 in communication with the hollow air passage 3221. The air incoming hole 3222 are in connection and communication with the air exhaustion port 312 of the air transfer pipeline 31. Multiple air spraying/spreading orifices 3223 are disposed on a sidewall of the armband-type terminal expansion spraying/spreading unit 322 in communication with the hollow air passage 3221. The air spraying/spreading orifices 3223 are directed to the body surface of the arm 42 of the user 4, whereby the compressed air in the hollow air passage 3221 can be sprayed and spread from the air spraying/spreading orifices 3223 toward the body surface of the arm 42 of the user 4 so as to spread the hot air layer 60 of the body surface of the user 4 and achieve double effects of final expansion and heat absorption.

Please refer to Figs. 6, 7-3, 8-1 and 8-2. The neck brace-type terminal expansion spraying/spreading unit 321 has multiple extension spraying pipeline connection ports 3217. The extension spraying pipeline connection ports 3217 are connectable with multiple extension spraying pipelines 3218. The extension spraying pipelines 3218 are further connected with the other terminal expansion spraying/spreading unit 32 on the other part of the body of the user 4, (such as the armband-type terminal expansion spraying/spreading unit 322, the mask-type terminal expansion spraying/spreading unit 324 or the helmet-type terminal expansion spraying/spreading unit 325 as shown in Fig. 6).

In the above embodiments of the present invention, the air compression unit 12 is a compressor with high compression ratio. Therefore, as shown in Figs. 2, 3 and 6, an air filtering member 111 can be used to filter the air to be taken into the air compression unit 12 before the air is taken into the air compression unit 12 (or to filter the compressed air after the compressed air is exhausted from the air compression unit 12) . Then, according to the physical phenomenon that the higher the air compression ratio is, the higher the temperature of the compressed air is, the instantaneous high temperature can be utilized to perform high-temperature harm reduction operation to the microparticles in the air, which are harmful to human body, (such as bacteria, dust mites, pollens, harmful microorganisms, allergens, etc.) After the harm reduction operation is performed to the compressed air, the compressed air expands to lower the temperature and restore to a pressure proximate to or equal to normal pressure. Thereafter, with the mask-type terminal expansion spraying/spreading unit 324 in communication with the air transfer pipeline 31, the air after the harm reduction operation is distributed to go through the mask-type terminal expansion spraying/spreading unit 324 for the mouth and nose 44 of the user 4 to breathe. Moreover, ultraviolet radiation can be additionally cooperatively used to enhance the harm reduction effect. Accordingly, in the condition the cost is not much increased, the requirement for medical level can be easily satisfied. Therefore, the present invention not only can provide cooling function, but also can be developed into medical quality application of air harm reduction. This is also a further design object of the present invention.

It can be known from the above cooling process of the present invention that all the air compression unit 12, the air heat dissipation passage set 13, the forced heat exchange heat dissipation unit 14, the power unit 15, the portable power supply device 2 and the compressed air transfer and terminal expansion spraying/spreading device 3 can have various structures and different assembling manners to achieve the same function. However, the present invention discloses two stressed design concepts totally different from the common cooling process . One the two stressed design concepts lies in that the heat of the compressed air is dissipated to lower the temperature in the transfer passage. The other of the two stressed design concepts lies in that the compressed air is directly sprayed to spread the hot air layer 60 on the body surface and perform the final expansion, heat absorption and temperature lowering. Therefore, in the condition the no traditional cooling means such as a coolant is used, the present invention can achieve cooling effect. In this case, the volume and weight of the cooling device can be greatly minified and the cooling and temperature lowering function can be more practically applied to a portable device.

According to the design of the above portable body surface air cooling device of the present invention, the present invention can truly directly operate the ambient air to effectively achieve cooling function for the body surface without using any coolant. The present invention improves the shortcomings of the conventional cooling device that the structure is complicated, the weight is heavy, the volume is huge and the use of the conventional cooling device fails to meet the requirement of environmental protection. Moreover, by means of the portable body surface air cooling device of the present invention, mobile cooling can be implemented in a safe environment without using any coolant. The present invention is truly a very practical design with high utility.

The above embodiments are only used to illustrate the present invention, not intended to limit the scope thereof. Many modifications of the above embodiments can be made without departing from the spirit of the present invention.

## Claims

1. A portable body surface air cooling method comprising steps of compressing air in an environment to form compressed air, transferring the compressed air through a transfer passage, dissipating the heat of the compressed air and lowering the temperature of the compressed air in the transfer process, making the compressed air with lowered temperature continuously self-expand in the transfer passage to reduce the pressure and lower the temperature, transferring the compressed air to a terminal expansion spraying/spreading position, directly spraying the compressed air to the body surface of a user so as to spread hot air layer on the body surface, and performing final expansion of the compressed air to absorb the heat and lower the temperature.

2. The portable body surface air cooling method as claimed in claim 1, further comprising a step of filtering the air in at least one of two situations before the air in the environment is compressed and after the air is compressed.

3. The portable body surface air cooling method as claimed in claim 1, wherein the air is compressed to form compressed air with higher temperature, whereby in the transfer passage, the compressed air with higher temperature transmits thermal energy to the transfer passage, which further transmits thermal energy to outer side so as to lower the temperature of the compressed air, also, externally added heat exchange heat dissipation operation being performed to the transfer passage from outer side of the transfer passage.

4. The portable body surface air cooling method as claimed in claim 1, wherein when the air in the environment with normal temperature is compressed into compressed air, the compressed air naturally generates high temperature, whereby the temperature is high enough to perform high-temperature harm elimination/reduction operation to microparticles in the air, which are harmful to human body.

5. The portable body surface air cooling method as claimed in any of claims 1 to 4, wherein the terminal expansion spraying/spreading position is for the user to breathe.

6. The portable body surface air cooling method as claimed in any of claims 1 to 5, wherein in at least one of the processes of heat dissipation and temperature lowering of the compressed air and self-expansion and pressure reduction and temperature lowering of the compressed air, the compressed air is transferred through an uninterrupted passage, which is fully free from affection of ambient environment temperature.

7. The portable body surface air cooling method as claimed in claim 1, comprising steps of:
(A) compressing air, the air in an environment being compressed into compressed air, which has such a physical property that in a normal environment, the compressed air can self-expand;
(B) dissipating the heat of the compressed air and lowering the temperature of the compressed air in the transfer process, the compressed air being transferred through a transfer passage, in the transfer process, the heat of the compressed air being dissipated and the temperature of the compressed air being lowered to lower the temperature of the compressed air;
(C) making the compressed air with lowered temperature continuously self-expand in the transfer passage heat-insulated from outer side thereof to reduce the pressure and lower the temperature, the compressed air with lowered temperature being continuously transferred through the transfer passage, whereby in the transfer process, the compressed air continuously self-expands to reduce the pressure and lower the temperature; and
(D) spraying and spreading the compressed air, which has self-expanded to reduce the pressure and lower the temperature toward the body surface of the user and performing final expansion and heat absorption, the compressed air that has self-expanded to reduce the pressure and lower the temperature in step C being directly sprayed and spread toward the body surface of the user so as to spread the hot air layer near the body surface, also, in the spraying/spreading process, the compressed air being made to perform final expansion and heat absorption effect so as to lower the temperature near the body surface and achieve double cooling effects.

8. A portable body surface air cooling device comprising a portable air compression and heat dissipation set (1), a portable power supply device (2) and a compressed air transfer and terminal expansion spraying/spreading device (3), the portable air compression and heat dissipation set (1) being connected with the compressed air transfer and terminal expansion spraying/spreading device (3), through the compressed air transfer and terminal expansion spraying/spreading device (3), the portable air compression and heat dissipation set (1) being connected with an air passage near a body surface position of a user (4), the portable air compression and heat dissipation set (1) being composed of a case (11), an air compression unit (12), an air heat dissipation passage set (13) and a power unit (15), the portable power supply device (2) serving to supply power for the power unit (15), whereby after the power unit (15) is operated, the air compression unit (12) is driven to compress the air in an environment and input the compressed air into the air heat dissipation passage set (13), after the heat of the compressed air is dissipated to lower the temperature, the compressed air being further transferred to the body surface position of the user (4) via the compressed air transfer and terminal expansion spraying/spreading device (3) to perform spraying/spreading and terminal expansion.

9. The portable body surface air cooling device as claimed in claim 8, wherein an externally added heat exchange heat dissipation unit (14) is assembled with the air heat dissipation passage set (13) to enhance the heat dissipation effect of the air heat dissipation passage set (13).

10. The portable body surface air cooling device as claimed in claim 9, wherein the air compression unit (12) has an air intake (121) and a compressed air exhaustion port (122), the air heat dissipation passage set (13) having an air heat dissipation passage air inlet (131) and an air heat dissipation passage air outlet (132), the air heat dissipation passage set (13) being at least composed of an air heat dissipation passage main body and air heat dissipation passage tunnels (135) integrally formed in the air heat dissipation passage main body (133), in addition, by means of the externally added heat exchange heat dissipation unit (14), the heat dissipation and temperature lowering process of the air heat dissipation passage main body (133) being enhanced, two ends of a compressed air conduit (123) being respectively connected with the compressed air exhaustion port (122) and the air heat dissipation passage air inlet (131), the compressed air transfer and terminal expansion spraying/spreading device (3) having an air transfer pipeline (31) for the air to flow through, the air transfer pipeline (31) being heat-insulated from outer side thereof and having at least one intake port (311) and an air exhaustion port (312) in connection and communication with the terminal expansion spraying/spreading unit (32), the intake port (311) being connected with the air heat dissipation passage air outlet (132).

11. The portable body surface air cooling device as claimed in claim 8, wherein an air filtering member (111) is additionally disposed to filter the air taken into the air compression unit in at least one of two situations before the air is taken into the air compression unit (12) and after the air is compressed.

12. The portable body surface air cooling device as claimed in claim 8, wherein the compressed air transfer passage of at least one of the air heat dissipation passage set (13) and the compressed air transfer and terminal expansion spraying/spreading device (3) is an uninterrupted transfer passage, which is fully free from affection of ambient environment temperature.

13. The portable body surface air cooling device as claimed in any of claims 8 to 12, wherein the compressed air transfer and terminal expansion spraying/spreading device (3) is assembled and connected with an air passage to a position near the body surface of the user (4) in communication with at least one terminal expansion spraying/spreading unit (32), in accordance with different application parts of the user (4), the terminal expansion spraying/spreading unit (32) being designed with various forms including at least one of a neck brace-type terminal expansion spraying/spreading unit (321) wearable on the neck (41) of the user (4), an armband-type terminal expansion spraying/spreading unit (322) wearable on the arm (42) of the user (4), a lower waist-type terminal expansion spraying/spreading unit (323) for spraying low-temperature compressed air to the limbs (43) below the waist of the user (4), a mask-type terminal expansion spraying/spreading unit (324) wearable on the mouth and nose (44) of the user (4) and a helmet-type terminal expansion spraying/spreading unit (325) wearable on the head (45) of the user (4).

14. The portable body surface air cooling device as claimed in claim 13, wherein the neck brace-type terminal expansion spraying/spreading unit (321) has at least one extension spraying pipeline connection ports (3217), the extension spraying pipeline connection port (3217) being connected with an extension spraying pipelines (3218), the extension spraying pipeline (3218) being further connected with the other terminal expansion spraying/spreading units (32).

15. The portable body surface air cooling device as claimed in any of claims 9 to 14, wherein the portable air compression and heat dissipation set (1) and the portable power supply device (2) are mounted on a wearable set carrier body (5) carried on the body of the user, the wearable set carrier body (5) being at least one of a waistband-type carrier body (51), a backpack-type carrier body (52) and a clothes-type carrier body, the backpack-type carrier body (52) has internal spaces (5210, 5220) for receiving the portable air compression and heat dissipation set (1) and the portable power supply device (2), the backpack-type carrier body (52) being designed with backing face air transfer passages (5211, 5221) and back strap air transfer passages (5212, 5222) in those parts in contact with the user (4), multiple air spraying/spreading orifices (52111, 52121, 52211, 52221) being formed on the backing face air transfer passages (5211, 5221) and the back strap air transfer passages (5212, 5222).
